# EUROPÄISCHE PATENTSCHRIFT

(11) **EP 4 142 807 B1**
(45) Veröffentlichungstag und Bekanntmachung des Hinweises auf die Patenterteilung: **25.09.2024**
(21) Anmeldenummer: 21722814.7
(22) Anmeldetag: 28.04.2021
(51) Int. Cl.: A61L 2/00, A61L 2/24, A61N 5/06

(54) **VORRICHTUNG UND VERFAHREN ZUR DESINFEKTION DER HÄNDE EINER PERSON**
APPARATUS AND METHOD FOR DISINFECTING THE HANDS OF A PERSON
APPAREIL ET PROCÉDÉ DE DÉSINFECTION DES MAINS D'UNE PERSONNE

(30) Priorität: 29.04.2020 DE 102020111635
(43) Veröffentlichungstag der Anmeldung: 08.03.2023
(73) Patentinhaber: Maier Werkzeugmaschinen GmbH & Co. KG, 78564 Wehingen (DE)
(72) Erfinder: SCHMALZRIED, Siegfried, 78224 Singen (DE); MAIER, Michael, 78564 Wehingen (DE)
(74) Vertreter: Schmid, Wolfgang
(86) Internationale Anmeldenummer: PCT/EP2021/061095
(87) Internationale Veröffentlichungsnummer: WO 2021/219701

(56) Entgegenhaltungen:
- DE-A1- 102017 009 637
- DE-A1- 102017 201 441
- US-A1- 2015 151 012
- US-A1- 2018 207 302

## Beschreibung

Die Erfindung betrifft eine Vorrichtung zur Desinfektion der Hände einer Person, mit einem Gehäuse, das einen zur Aufnahme wenigstens einer Hand geeigneten Hohlraum aufweist. Des Weiteren betrifft die Erfindung ein Verfahren zum Betreiben einer solchen Vorrichtung.

Die gesamte menschliche Bevölkerung ist seit Beginn des Jahres 2020 der Covid-19-Pandemie, umgangssprachlich auch als Corona-Virus-Pandemie oder ähnliches bezeichnet, ausgesetzt. Unabhängig von der Covid-19-Pandemie kommt es jährlich weltweit zu einer großen Anzahl an Fällen von Influenza bzw. Virusgrippe. Obwohl die Influenza bereits seit mehreren Jahrhunderten als Erkrankung bekannt ist, besteht auch gegen diese Krankheit bislang kein wirkungsvoller Schutz.

Die Verbreitung der Viren erfolgt auf die unterschiedlichsten Arten, neben der Übertragung durch die Atemluft auch durch Kontakt der Viren mit der Haut, insbesondere mit den Händen, von wo sie auf die Schleimhäute und dadurch in den Körper gelangen können.

Um diese Art der Übertragung zu vermeiden, wird ein häufiges Händewaschen empfohlen. Dies ist, abhängig davon wie der Waschvorgang ausgeführt wird, jedoch zum einen nicht der sicherste Weg, um die Viren von der Haut zu entfernen, zum anderen führt ein häufiges Händewaschen zum Austrocknen der Haut.

Aus dem allgemeinen Stand der Technik sind Vorrichtungen zum Ausgeben von UV-Strahlung bekannt, deren äußere Form derjenigen einer Taschenlampe ähnelt, und mit denen zum Beispiel die Hände desinfiziert werden können. Problematisch dabei ist jedoch, dass eine zu lange Einwirkzeit und damit eine zu hohe Dosis der UV-Strahlung auf der Haut zu Hautkrankheiten bis hin zu Hautkrebs führen kann.

Die EP 2 465 543 A1 beschreibt eine Vorrichtung zur Sterilisation oder Desinfektion der Hände einer Person, mit einem mit mindestens einer Eintrittsöffnung zum Einführen der Hände versehenen Gehäuse und mit innerhalb des Gehäuse angeordneten, UV-Licht emittierenden Mitteln zur Bestrahlung einer innerhalb des Gehäuse angeordneten Bestrahlungszone. Das Gehäuse weist mit einer reflektierenden Oberfläche zur Reflexion von UV-Licht versehene Innenwände auf, die durch eine Beschichtung aus Titandioxid-Nanopartikel gebildet ist.

Bei derartigen, mit Spiegeln oder reflektierenden Schichten zur Weiterleitung der Strahlung versehenen Vorrichtungen besteht ein erheblicher Nachteil in der Entstehung eines Schattens, der die Funktionsweise der Vorrichtung erheblich beeinträchtigen kann.

Aus der US 2002/0122743 A1 sind ein Verfahren und eine Vorrichtung zum Sterilisieren eines Objekts in einem Mehrzweck-Haushaltsgerät wie Mikrowellenherden, Geschirrspülern, Waschmaschinen usw. bekannt. Die Vorrichtung umfasst eine Gehäuseeinheit mit einer Kammer zur Aufnahme des zu sterilisierenden Objekts, eine außerhalb der Kammer positionierte UV-Lichtquelle und eine Belichtungsplatte mit festem, einziehbarem oder aufklappbarem Design.

Für eine schnelle und auch von unerfahrenen Nutzern leicht verständliche Desinfektion der Hände einer Person ist diese Vorrichtung jedoch nicht geeignet.

Die DE 10 2017 201 441 A1 beschreibt eine gattungsgemäße Vorrichtung und ein gattungsgemäßes Verfahren.

Die US 2015/151012 A1 betrifft eine Vorrichtung und ein Verfahren zur Desinfektion von Hautbereichen, insbesondere zur Desinfektion von Händen. Die Desinfektion erfolgt durch die Anwendung eines kurzen IR-Lichtimpulses, der von mindestens einer IR-Quelle auf den Hautbereich abgegeben wird. Die Parameter des Lichtimpulses werden so gewählt, dass Keime an der Hautoberfläche abgetötet werden und gleichzeitig eine Schädigung tieferer Hautschichten verhindert wird.

Die DE 10 2017 009 637 A1 beschreibt einen Handtrockner mit einem Gehäuse, in dem ein durch eine Gehäuseöffnung außen zugänglicher Hohlraum zur Aufnahme mittels einer Luftströmung im Hohlraum zu trocknender Hände ausgebildet ist, und mit einer Vorrichtung zur Erzeugung der Luftströmung sowie einer Vorrichtung zur Erzeugung von UV-Strahlung, welche wenigstens eine im ultravioletten Wellenlängenbereich emittierende Lampe umfasst.

Die US 2018/207302 A1 betrifft ein Verfahren und eine Vorrichtung zur Verminderung von Mikroben, die den Einsatz von Nahinfrarotlicht (NIR), UV-, violett und blau emittierenden LED-Elementen sowie OLED umfassen.

Es ist Aufgabe der vorliegenden Erfindung, eine Vorrichtung und ein Verfahren zur Desinfektion der Hände einer Person zu schaffen, die einerseits eine zuverlässige Desinfektion gewährleisten und andererseits sicherstellen, dass durch die Anwendung derselben keine Hauterkrankungen oder ähnliches auftreten können.

Erfindungsgemäß wird diese Aufgabe durch die in Anspruch 1 genannten Merkmale gelöst.

Die erfindungsgemäße Vorrichtung ist durch den Einsatz von Lichtstrahlung im UV- und/oder IR-Bereich in der Lage, eine zuverlässige Desinfektion der Hände einer Person zu ermöglichen. Der besondere Vorteil der erfindungsgemäßen Lösung liegt dabei darin, dass es sich durch die Verwendung der Lichtstrahlung um eine berührungslose Desinfektion handelt, für deren Anwendung der Benutzer keine Vorkenntnisse benötigt. Vielmehr wird der Desinfektionsvorgang sozusagen automatisch zuverlässig ausgeführt.

Im Gegensatz zum üblichen Händewaschen erfordert die erfindungsgemäße Vorrichtung keinerlei Einsatz von Verbrauchsmaterialien, so dass sie stets einsatzbereit ist und auch vergleichsweise kostengünstig betrieben werden kann. Damit ist die erfindungsgemäße Lösung insbesondere, jedoch nicht nur, für öffentliche Einrichtungen, wie Krankenhäuser, Pflegeheime und dergleichen, geeignet.

Die erfindungsgemäße Steuereinrichtung ist dabei in der Lage, eine Steuerung der Lichtquelle und/oder der Ausgabeeinrichtung und/oder der Verfahreinrichtung vorzunehmen, so dass eine Überbelastung der Haut vermieden werden kann. Dabei ermöglicht die Verfahreinrichtung ein Bewegen der Lichtstrahlung innerhalb des Hohlraums, wodurch verhindert werden kann, dass die Lichtstrahlung zu lange auf einen Bereich der Hände gerichtet bleibt. Dies stellt gegenüber sämtlichen bekannten Lösungen einen erheblichen Vorteil dar, da die erfindungsgemäße Vorrichtung dadurch völlig bedenkenlos und auch mehrmals täglich benutzt werden kann.

Dadurch, dass die Steuereinrichtung dafür vorgesehen ist, die wenigstens eine Lichtquelle und/oder die wenigstens eine Ausgabeeinrichtung und/oder die wenigstens eine Verfahreinrichtung derart zu steuern, dass die auf die Hände ausgegebene Intensität und/oder Dosis der Lichtstrahlung eine festgelegte Obergrenze nicht überschreitet, wird eine Überbelastung der Haut zuverlässig vermieden, so dass weder Krankheiten noch andere Schädigungen der Haut des Benutzers auftreten können.

Erfindungsgemäß ist des Weiteren vorgesehen, dass die Steuereinrichtung dafür vorgesehen ist, die Verfahrgeschwindigkeit der Verfahreinrichtung in Abhängigkeit von der festgelegten Obergrenze einzustellen, dass der Abstand der wenigstens einen Hand von der wenigstens einen Ausgabeeinrichtung mittels einer Messeinrichtung messbar ist, und dass die Steuereinrichtung dafür vorgesehen ist, die Leistung der Lichtquelle in Abhängigkeit von dem gemessenen Abstand einzustellen. Dies trägt ebenfalls dazu bei, die auf die Hände einwirkende Strahlenbelastung zu verringern.

Eine alternative Lösung der Aufgabe ergibt sich aus Anspruch 2.

Erfindungsgemäß ist dabei eine Sensoreinrichtung zur Überwachung der Position der Hände vorgesehen, die mit der Steuereinrichtung in Wirkverbindung steht, um die jeweilige Position der Hände and die Steuereinrichtung weiterzuleiten, wobei die Steuereinrichtung dafür vorgesehen ist, die Verfahreinrichtung derart zu steuern, dass Änderungen der Position der Hände durch Verfahren der Ausgabeeinrichtung mittels der Verfahreinrichtung ausgeglichen werden. Dadurch lassen sich Änderungen der Position der Hände auf einfache Weise durch ein Verfahren der Ausgabeeinrichtung mittels der Verfahreinrichtung ausgleichen.

Ein sicheres Verfahren der Ausgabeeinrichtung innerhalb des Gehäuses, um auf diese Weise die Lichtstrahlung innerhalb des Hohlraums zu bewegen, ergibt sich, wenn die Verfahreinrichtung wenigstens ein mit der wenigstens einen Ausgabeeinrichtung gekoppeltes Führungselement aufweist.

Um eine effektive Desinfektion der Hände bzw. wenigstens einer Hand zu erreichen, können des Weiteren zwei Ausgabeeinrichtungen auf gegenüberliegenden Seiten des Gehäuses vorgesehen sein.

Des Weiteren kann vorgesehen sein, dass das Gehäuse mit Ausnahme einer Öffnung zum Einführen wenigstens einer Hand allseitig geschlossen ist. Dadurch wird zum einen für den Benutzer deutlich, wo er seine Hände zur Nutzung der erfindungsgemäßen Vorrichtung einführen muss, und es wird zum anderen sichergestellt, dass die ausgegebene UV- oder IR-Strahlung innerhalb des Gehäuses verbleibt.

Als im Hinblick auf die Desinfektionswirkung der erfindungsgemäßen Vorrichtung besonders vorteilhaft hat es sich herausgestellt, wenn die Wellenlänge der Lichtstrahlung 210 bis 230 nm, vorzugsweise 215 bis 225 nm, und/oder 800 bis 900 nm, vorzugsweise 830 bis 870 nm, beträgt.

In einer weiteren vorteilhaften Ausgestaltung der Erfindung kann vorgesehen sein, dass die Lichtquelle eine Leistung von 20 bis 40 mW aufweist. Eine solche Leistung der Lichtquelle stellt einerseits eine wirkungsvolle Desinfektion sicher und verhindert andererseits Hautschädigungen oder ähnliches.

Ein Kompromiss aus einer kurzen Desinfektionszeit und einer kostengünstigen Vorrichtung ergibt sich, wenn die Verfahreinrichtung eine Verfahrgeschwindigkeit von 1 bis 3 m/min, vorzugsweise 1,5 bis 2,5 m/min, aufweist.

In Anspruch 9 ist ein Verfahren zum Betreiben einer erfindungsgemäßen Vorrichtung angegeben.

Das erfindungsgemäße Verfahren ermöglicht eine schnelle und sichere Desinfektion der Hände, so dass ein Benutzer auf ein bislang notwendiges, häufiges Händewaschen verzichten kann. Dies bringt dem Benutzer nicht nur Zeitvorteile, sondern verringert auch die Kosten für Verbrauchsmaterial, wie Seife, Wasser und Handtücher oder ähnliches.

Dabei wird durch die erfindungsgemäße Steuerung der Lichtquelle und/oder der Ausgabeeinrichtung und/oder der Verfahreinrichtung durch die Steuerungseinrichtung ein Schutz des Benutzers vor einer Überbelastung der Haut vermieden.

Dabei ist vorgesehen, dass die Steuereinrichtung die Verfahrgeschwindigkeit der Verfahreinrichtung in Abhängigkeit von der festgelegten Obergrenze einstellt. Auch durch ein entsprechend schnelles Bewegen der Verfahreinrichtung kann eine Überbelastung der Haut vermieden werden, wobei gleichzeitig die Geschwindigkeit so niedrig gewählt werden sollte, dass eine zuverlässige Desinfektion der Hände gewährleistet ist.

Eine alternative Lösung der Aufgabe ergibt sich aus Anspruch 10.

Dabei ist vorgesehen, dass der Abstand der wenigstens einen Hand von der wenigstens einen Ausgabeeinrichtung mittels einer Messeinrichtung gemessen wird, und dass die Steuereinrichtung die Leistung der Lichtquelle in Abhängigkeit von dem gemessenen Abstand einstellt. Eine Verringerung der Leistung der Lichtquelle in Abhängigkeit des Abstands der Hände von der Ausgabeeinrichtung ist insofern vorteilhaft, als dadurch die jeweils erforderliche Lichtleistung auf die zu desinfizierenden Hände aufgebracht wird.

Eine alternative Lösung der Aufgabe ergibt sich aus Anspruch 11.

Um eventuelle, durch Bewegungen hervorgerufene Änderungen der Position der Hände auszugleichen, ist dabei vorgesehen, dass die Steuereinrichtung die Verfahreinrichtung in Abhängigkeit einer mittels einer Sensoreinrichtung ermittelten Position der Hände derart steuert, dass Änderungen der Position der Hände durch Verfahren der Ausgabeeinrichtung mittels der Verfahreinrichtung ausgeglichen werden.

Eine einfache Möglichkeit zur Begrenzung der Dosis der Lichtstrahlung ergibt sich, wenn die Steuereinrichtung die Leistung der Lichtquelle in Abhängigkeit von der festgelegten Obergrenze einstellt.

Alternativ oder zusätzlich kann auch vorgesehen sein, dass die Steuereinrichtung die Ausgabe der Lichtstrahlung durch die Ausgabeeinrichtung in Abhängigkeit von der festgelegten Obergrenze zeitlich begrenzt. Auch auf diese Weise lässt sich eine Überbeanspruchung der Haut zuverlässig vermeiden.

Nachfolgend ist ein Ausführungsbeispiel der Erfindung anhand der Zeichnung prinzipmäßig dargestellt.

Es zeigt:
- Fig. 1: eine perspektivische Ansicht der erfindungsgemäßen Vorrichtung;
- Fig. 2: eine Explosionsdarstellung der erfindungsgemäßen Vorrichtung aus Fig. 1;
- Fig. 3: eine Vorderansicht der erfindungsgemäßen Vorrichtung;
- Fig. 4: eine Seitenansicht der erfindungsgemäßen Vorrichtung;
- Fig. 5: eine weitere perspektivische Explosionsansicht der erfindungsgemäßen Vorrichtung; und
- Fig. 6: eine weitere Ausführungsform der erfindungsgemäßen Vorrichtung.

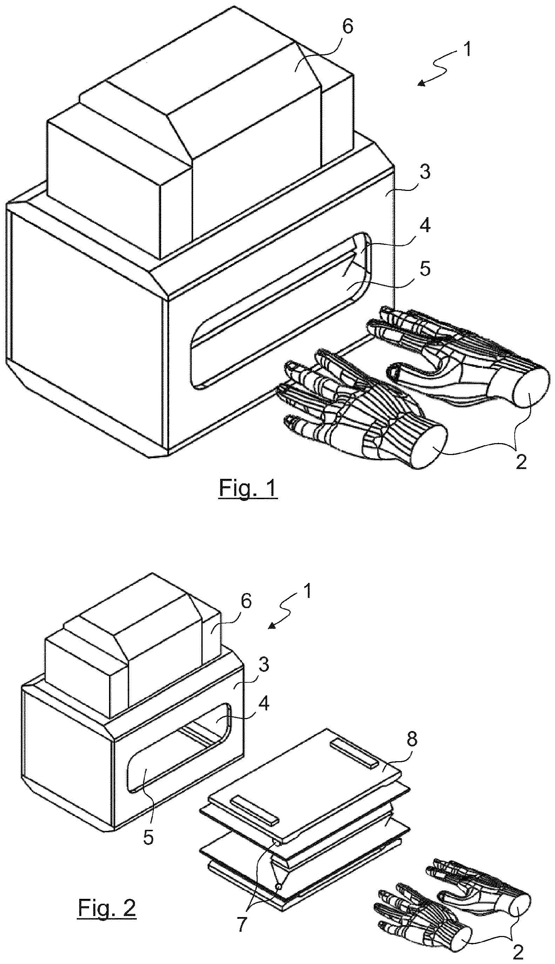

Die Figuren 1 bis 6 zeigen eine Vorrichtung 1 zur Desinfektion der Hände 2 einer nicht dargestellten Person. Die Vorrichtung 1 weist ein Gehäuse 3 auf, das wiederum einen zur Aufnahme wenigstens einer Hand, im vorliegenden Fall beider Hände 2, geeigneten Hohlraum 4 aufweist. Das Gehäuse 3 ist mit Ausnahme einer Öffnung 5 zum Einführen wenigstens einer der Hände 2 allseitig geschlossen. Im vorliegenden Fall befindet sich die Öffnung 5 an einer Vorderseite der Vorrichtung 1 und ist als Schlitz ausgeführt. Grundsätzlich sind jedoch auch andere Möglichkeiten zur Anordnung und zur Ausführung der Öffnung 5 an dem Gehäuse 3 denkbar. Des Weiteren ist es selbstverständlich möglich, die Vorrichtung 1 auf eine solche Weise an einer Wand oder an einem anderen geeigneten Ort zu montieren, dass sich die Öffnung 5 oben, seitlich oder unten befindet. Die dargestellte Größe des Gehäuses 3 ist als rein beispielhaft anzusehen; diese kann an den jeweiligen Anwendungszweck angepasst werden.

Die Vorrichtung 1 weist des Weiteren eine Lichtquelle 6 auf, die zur Erzeugung einer gebündelten bzw. fokussierten Lichtstrahlung im UV- und/oder IR-Bereich dient. Die Wellenlänge der von der Lichtquelle 6 erzeugten Lichtstrahlung kann beispielsweise in einem Bereich von 210 bis 230 nm, vorzugsweise 215 bis 225 nm und besonders bevorzugt 220 bis 222 nm, betragen. In diesem Fall liegt die Lichtstrahlung im ultravioletten Bereich bzw. UV-Bereich. Alternativ oder zusätzlich ist es möglich, mit der Lichtquelle 6 eine Wellenlänge der Lichtstrahlung von 800 bis 900 nm, vorzugsweise 830 bis 870 nm und besonders bevorzugt 850 nm, zu erzeugen. Die Lichtstrahlung liegt in diesem Fall im infraroten Bereich bzw. IR-Bereich. Auch die gleichzeitige Erzeugung von Lichtstrahlung in den beiden Bereichen, also in dem UV-Bereich und in dem IR-Bereich, ist denkbar.

Im dargestellten Ausführungsbeispiel ist die Lichtquelle 6 an der Oberseite des Gehäuses 3 montiert. Es wäre jedoch auch möglich, die Lichtquelle 6 in einem anderen Bereich des Gehäuses 3 anzubringen, gegebenenfalls auch an einem von dem Gehäuse 3 entfernten Ort.

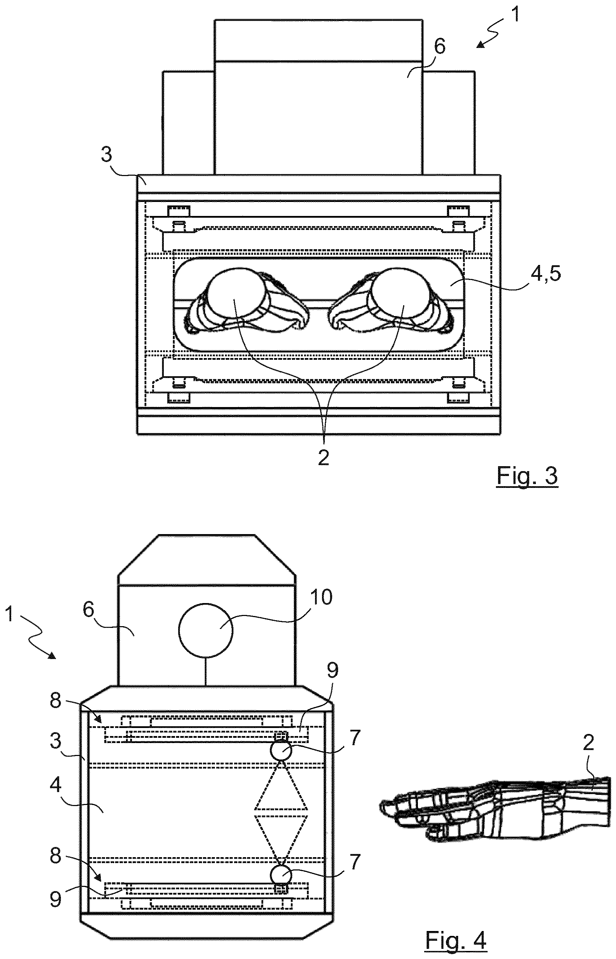

Die Lichtquelle 6 steht mit einer in Fig. 4 am besten zu erkennenden Ausgabeeinrichtung 7 in Wirkverbindung, die zur Ausgabe der Lichtstrahlung in den Hohlraum 4 dient. Bei der Ausgabeeinrichtung 7 handelt es sich insbesondere um eine optische Einrichtung, die Linsen, Spiegel oder dergleichen aufweisen kann. Die Verbindung zwischen der Lichtquelle 6 und der Ausgabeeinrichtung 7 kann beispielsweise mittels geeigneter Lichtleiter realisiert werden. Wie in Fig. 4 zu erkennen ist, sind im vorliegenden Fall zwei auf gegenüberliegenden Seiten des Gehäuses 3 angeordnete Ausgabeeinrichtungen 7 vorgesehen. Dadurch können die durch die Öffnung 5 in den Hohlraum 4 eingeführten Hände 2 sowohl an ihrer Oberseite als auch an ihrer Unterseite mit der Lichtstrahlung beaufschlagt werden. Die Lichtstrahlung ist in Fig. 4 mittels den gestrichelten, ein Dreieck bildenden Linien angedeutet, d.h. der von der Ausgabeeinrichtung 7 ausgegebene Lichtstrahl ist aufgeweitet bzw. divergent ausgeführt, sodass der mit Licht beaufschlagte Bereich vergrößert ist. Diese Aufweitung bzw. Divergenz kann variabel sein, um die Strahlungsintensität bzw. -dosis in dem beaufschlagten Bereich zu variieren.

Die Vorrichtung 1 weist des Weiteren wenigstens eine Verfahreinrichtung 8 auf, die zum Bewegen der Lichtstrahlung innerhalb des Hohlraums 4 dient. Dadurch kann die Lichtstrahlung gleichmäßig innerhalb des Hohlraums 4 verteilt werden, so dass die Hände 2 gleichmäßig und gründlich desinfiziert werden.

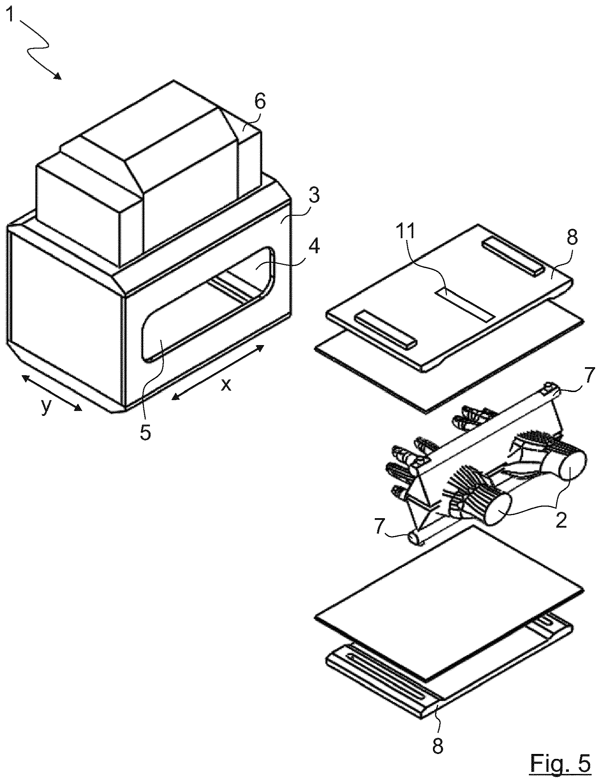

Im vorliegenden Ausführungsbeispiel weist die Verfahreinrichtung 8 jeweilige, mit den beiden Ausgabeeinrichtungen 7 gekoppelte Führungselemente 9 auf. Bei den Führungselementen 9 kann es sich um Schlitten oder ähnliches handeln, mit denen die Ausgabeeinrichtungen 7 innerhalb des Gehäuses 3 bewegt werden können. Das Verfahren der Ausgabeeinrichtungen 7 innerhalb des Gehäuses 3 der Vorrichtung 1 kann dabei über eine parallel zu der Einführungsrichtung der Hände 2 verlaufende, also horizontal ausgerichtete Ebene erfolgen, die durch die in Fig. 5 mit "x" und "y" bezeichneten Richtungen definiert ist. Hierbei ist es möglich, die Ausgabeeinrichtung 7 beispielsweise in der y-Richtung von der Öffnung 5 weg und zu derselben zurück zu bewegen, wenn, wie im vorliegenden Fall, die Lichtstrahlung in Form eines Vorhangs von der Ausgabeeinrichtung 7 ausgegeben wird. Alternativ ist es auch möglich, die Lichtstrahlung im Prinzip punktförmig auszugeben und die Ausgabeeinrichtung 7 sowohl in der mit x- als auch in der y-Richtung, d.h. über die komplette xy-Ebene zu bewegen.

Im dargestellten Ausführungsbeispiel bewegt die Verfahreinrichtung 8 die Ausgabeeinrichtungen 7 also in einer Richtung, die im Wesentlichen senkrecht zu der Richtung verläuft, in der sich die Ausgabeeinrichtungen 7 erstrecken. Während sich die Ausgabeeinrichtungen 7 in x-Richtung erstrecken, bewegt die Verfahreinrichtung 8 die Ausgabeeinrichtungen 7 in der y-Richtung. Dadurch wird die gesamte Fläche innerhalb des Hohlraums 4 mit der Lichtstrahlung beaufschlagt und es können gegenüber einer Lösung, in der die Ausgabeeinrichtungen 7 über die gesamte Fläche des Hohlraums 4 angeordnet sind, Kosten eingespart werden. Selbstverständlich wäre es auch möglich, die Ausgabeeinrichtungen 7 in y-Richtung verlaufend anzuordnen und dieselben mittels der Verfahreinrichtung 8 in x-Richtung zu bewegen.

Des Weiteren ist es möglich, das Bewegen der Lichtstrahlung innerhalb des Hohlraums 4 durch Spiegel oder ähnliche optische Elemente zu erreichen, wobei die Ausgabeeinrichtung 7 in diesem Fall stationär bleibt und nicht bewegt wird. Auch ein Verkippen der Ausgabeeinrichtung 7 um eine Achse zum Bewegen der Lichtstrahlung ist denkbar.

Die Vorrichtung 1 weist des Weiteren eine nur in Fig. 4 sehr schematisch angedeutete Steuereinrichtung 10 auf, die zur Steuerung der Lichtquelle 6 und/oder der Ausgabeeinrichtung 7 und/oder der Verfahreinrichtung 8 dient. Die Steuereinrichtung 10 ist dafür vorgesehen, die Lichtquelle 6 und/oder die Ausgabeeinrichtung 7 und/oder die Verfahreinrichtung 8 derart zu steuern, dass die auf die Hände 2 ausgegebene Intensität und/oder Dosis der Lichtstrahlung eine festgelegte Obergrenze nicht überschreitet. Diese Obergrenze kann nach medizinischen Gesichtspunkten festgelegt werden. Gegebenenfalls kann es möglich sein, die Obergrenze auch zu verändern bzw. an bestimmte Gegebenheiten anzupassen.

Zum Beispiel kann die Steuereinrichtung 10 die Leistung der Lichtquelle 6, die im vorliegenden Fall vorzugsweise 20 bis 40 mW, beispielsweise 30 mW, beträgt, in Abhängigkeit von der festgelegten Obergrenze einstellen. Alternativ oder zusätzlich kann die Steuereinrichtung 10 die Ausgabe der Lichtstrahlung durch die Ausgabeeinrichtung 7 in Abhängigkeit von der festgelegten Obergrenze zeitlich begrenzen.

Des Weiteren ist es möglich, zusätzlich die Verfahrgeschwindigkeit der Verfahreinrichtung 8 durch die Steuerung der Steuereinrichtung 10 in Abhängigkeit von der festgelegten Obergrenze einzustellen. Diese Verfahrgeschwindigkeit der Verfahreinrichtung 8 beträgt im vorliegenden Fall vorzugsweise 1 bis 3 m/min, noch bevorzugter 1,5 bis 2,5 m/min. Durch ein schnelleres Bewegen der Lichtstrahlung innerhalb des Hohlraums 4 wird die Dosis der auf die Hände 2 aufgebrachten Lichtstrahlung verringert bzw. umgekehrt wird durch ein langsameres Bewegen der Lichtstrahlung die Dosis erhöht.

Eine weitere Möglichkeit zur Beeinflussung der auf die Hände 2 bzw. auf die wenigstens einen Hand 2 aufgebrachten Lichtstrahlung besteht darin, den Abstand der wenigstens einen Hand 2 von den Ausgabeeinrichtungen 7 mittels einer nicht dargestellten Messeinrichtung zu messen und die Leistung der Lichtquelle 6 in Abhängigkeit von dem gemessenen Abstand mittels der Steuereinrichtung 10 einzustellen. Dabei wird die Leistung der Lichtquelle 6 am Punkt des Auftreffens der Lichtstrahlung auf die Hände 2 eingestellt. Dieses Messen und Einstellen der Leistung der Lichtquelle 6 kann auch in einen Regelmechanismus eingebunden werden, so dass der Abstand ständig gemessen und die Leistung ständig eingestellt wird.

Die Vorrichtung 1 weist des Weiteren eine in Fig. 5 nur schematisch angedeutete optische Sensoreinrichtung 11 auf. Mittels der in den Hohlraum 4 gerichteten Sensoreinrichtung 11 ist es möglich, die Position der Hände 2 zu überwachen. Die Sensoreinrichtung 11 steht mit der Steuereinrichtung 10 in Wirkverbindung, sodass die jeweilige Position der Hände 2 and die Steuereinrichtung 10 weitergeleitet werden kann. Dadurch ist die Steuereinrichtung 10 in der Lage, die Verfahreinrichtung 8 derart zu steuern, dass etwaige Änderungen der Position der Hände 2 durch entsprechendes Verfahren der Ausgabeeinrichtung 7 mittels der Verfahreinrichtung 8 ausgeglichen werden. Auf diese Weise entsteht ein Regelkreis aus der Sensoreinrichtung 11, der Steuereinrichtung 10 und der Verfahreinrichtung 8. Dadurch wird eine gleichmäßige Bestrahlung der Hände 2 erreicht und insbesondere eine bereichsweise zu starke Bestrahlung derselben vermieden, was ansonsten, beispielsweise bei einer zufälligen Bewegung der Hände und der Verfahreinrichtung 8 in dieselbe Richtung, der Fall sein könnte. Im Prinzip handelt es dabei um eine Überwachung der durch die Vorrichtung 1 erzeugten Strahlenbelastung.

Des Weiteren kann die Vorrichtung 1 eine nicht dargestellte optische oder akustische Anzeigeeinrichtung aufweisen, die anzeigt, wenn der Desinfektionsvorgang beendet ist.

Außerdem kann die Steuereinrichtung 10 optional in der Lage sein, zu erfassen, welche Personen wann ihre Hände desinfiziert haben. Hierzu kann die Steuereinrichtung 10 mit einer für diesen Zweck geeigneten Einrichtung verbunden sein, die die entsprechenden Daten der Personen erfasst. Auf diese Weise kann zum Beispiel sichergestellt werden, dass alle Mitarbeiter in einem kritischen Bereich ihre Hände desinfiziert haben. Auch eine Kopplung der Vorrichtung 1 mit Zeiterfassungseinrichtungen oder eine Integration von Zeiterfassungseinrichtungen in die Vorrichtung 1 ist denkbar, um Mitarbeitern den Zutritt zu ihrer Arbeitsstätte erst dann zu ermöglichen, wenn sie ihre Hände desinfiziert haben.

Des Weiteren kann die Sensoreinrichtung 11 beispielsweise auch eingesetzt werden, um zu ermitteln, ob die betreffende Person ihre Hände ausreichend lange der Lichtstrahlung ausgesetzt hat. Auf diese Weise lässt sich die Vorrichtung 1 auch im Rahmen von Zutrittskontrollen in öffentlichen Gebäuden einsetzen, um eine ausreichende Hygiene von Besuchern sicherzustellen. Die Sensoreinrichtung 11 kann auch so ausgebildet sein, dass sie erkennt, ob sich tatsächlich eine Hand oder etwas vollkommen anderes in dem Hohlraum 4 befindet, sodass eine Umgehung der Vorrichtung 1 unterbunden werden kann.

Die oben beschriebene Sensoreinrichtung 11 kann auch zum Einschalten der Vorrichtung 1 bzw. der Lichtquelle 6 und gegebenenfalls der Steuereinrichtung 10 und damit auch der weiteren, oben beschriebenen Einrichtungen der Vorrichtung 1 verwendet werden, indem die Sensoreinrichtung 11 das Einführen der Hände 2 in den Hohlraum 4 erkennt und an die Steuereinrichtung 10 weiterleitet.

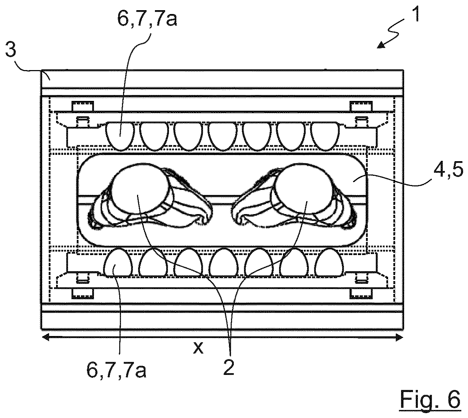

In einer in Fig. 6 dargestellten Ausführungsform der Vorrichtung 1 sind die Lichtquelle 6 und die Ausgabeeinrichtung 7 als eine verbundene Einheit ausgebildet. Dabei sind die Lichtquelle 6 und die Ausgabeeinrichtung 7 durch mehrere, in der Form eines Lichtbands angeordnete LEDs 7a gebildet. Im vorliegenden Fall weist die Lichtquelle 6 eine nicht näher dargestellte Leuchtdiode auf, während die Ausgabeeinrichtung 7 durch eine ebenfalls nicht dargestellte Verkapselung und/oder Fassung der Leuchtdiode ausgebildet ist. Die Verkapselung bzw. Ausgabeeinrichtung 7 weist hierbei optische Eigenschaften auf, um bei einem von der Leuchtdiode ausgehenden Licht geeignete Strahleigenschaften auszubilden. Die LEDs 7a sind ferner eingerichtet, das Licht in Richtung des Hohlraums 4 und somit in Richtung der in denselben eingeführten Hände 2 auszugeben. Auf diese Weise können Probleme bezüglich einer Schattenbildung vermieden werden. Die oben beschriebene Verfahreinrichtung 8 bewegt dabei das gesamte Lichtband, d.h. nicht nur die Ausgabeeinrichtung 7, sondern auch die Lichtquelle 6, sodass das gesamte, durch die LEDs 7a in x-Richtung gebildete Lichtband in der y-Richtung bewegt wird.

## Patentansprüche

1. Vorrichtung (1) zur Desinfektion der Hände (2) einer Person, mit einem Gehäuse (3), das einen zur Aufnahme wenigstens einer Hand (2) geeigneten Hohlraum (4) aufweist, mit wenigstens einer Lichtquelle (6) zur Erzeugung einer gebündelten Lichtstrahlung im UV- und/oder IR-Bereich, wenigstens eine mit der Lichtquelle (6) in Wirkverbindung stehende Ausgabeeinrichtung (7) zur Ausgabe der Lichtstrahlung in den Hohlraum (4), mit wenigstens einer Verfahreinrichtung (8) zum Bewegen der Lichtstrahlung und mit wenigstens einer Steuereinrichtung (10) zur Steuerung der Lichtquelle (6) und/oder der Ausgabeeinrichtung (7) und/oder der Verfahreinrichtung (8), wobei die Steuereinrichtung (10) dafür vorgesehen ist, die wenigstens eine Lichtquelle (6) und/oder die wenigstens eine Ausgabeeinrichtung (7) und/oder die wenigstens eine Verfahreinrichtung (8) derart zu steuern, dass die auf die Hände (2) ausgegebene Intensität und/oder Dosis der Lichtstrahlung eine festgelegte Obergrenze nicht überschreitet,
**dadurch gekennzeichnet, dass**
die Steuereinrichtung (10) dafür vorgesehen ist, die Verfahrgeschwindigkeit der Verfahreinrichtung (8) in Abhängigkeit von der festgelegten Obergrenze einzustellen, dass der Abstand der wenigstens einen Hand (2) von der wenigstens einen Ausgabeeinrichtung (7) mittels einer Messeinrichtung messbar ist, und dass die Steuereinrichtung (10) dafür vorgesehen ist, die Leistung der Lichtquelle (6) in Abhängigkeit von dem gemessenen Abstand einzustellen.

2. Vorrichtung (1) zur Desinfektion der Hände (2) einer Person, mit einem Gehäuse (3), das einen zur Aufnahme wenigstens einer Hand (2) geeigneten Hohlraum (4) aufweist, mit wenigstens einer Lichtquelle (6) zur Erzeugung einer gebündelten Lichtstrahlung im UV- und/oder IR-Bereich, wenigstens eine mit der Lichtquelle (6) in Wirkverbindung stehende Ausgabeeinrichtung (7) zur Ausgabe der Lichtstrahlung in den Hohlraum (4), mit wenigstens einer Verfahreinrichtung (8) zum Bewegen der Lichtstrahlung und mit wenigstens einer Steuereinrichtung (10) zur Steuerung der Lichtquelle (6) und/oder der Ausgabeeinrichtung (7) und/oder der Verfahreinrichtung (8), wobei die Steuereinrichtung (10) dafür vorgesehen ist, die wenigstens eine Lichtquelle (6) und/oder die wenigstens eine Ausgabeeinrichtung (7) und/oder die wenigstens eine Verfahreinrichtung (8) derart zu steuern, dass die auf die Hände (2) ausgegebene Intensität und/oder Dosis der Lichtstrahlung eine festgelegte Obergrenze nicht überschreitet,
**gekennzeichnet durch**
eine Sensoreinrichtung (11) zur Überwachung der Position der Hände (2), die mit der Steuereinrichtung (10) in Wirkverbindung steht, um die jeweilige Position der Hände (2) and die Steuereinrichtung (10) weiterzuleiten, wobei die Steuereinrichtung (10) dafür vorgesehen ist, die Verfahreinrichtung (8) derart zu steuern, dass Änderungen der Position der Hände (2) durch Verfahren der Ausgabeeinrichtung (7) mittels der Verfahreinrichtung (8) ausgeglichen werden.

3. Vorrichtung nach Anspruch 1 oder 2,
**dadurch gekennzeichnet, dass**
die Verfahreinrichtung (8) wenigstens ein mit der wenigstens einen Ausgabeeinrichtung (7) gekoppeltes Führungselement (9) aufweist.

4. Vorrichtung nach Anspruch 1, 2 oder 3,
**dadurch gekennzeichnet, dass**
zwei Ausgabeeinrichtungen (7) auf gegenüberliegenden Seiten des Gehäuses (3) vorgesehen sind.

5. Vorrichtung nach einem der Ansprüche 1 bis 4,
**dadurch gekennzeichnet, dass**
das Gehäuse (3) mit Ausnahme einer Öffnung (5) zum Einführen wenigstens einer Hand (2) allseitig geschlossen ist.

6. Vorrichtung nach einem der Ansprüche 1 bis 5,
**dadurch gekennzeichnet, dass**
die Wellenlänge der Lichtstrahlung 210 bis 230 nm, vorzugsweise 215 bis 225 nm, und/oder 800 bis 900 nm, vorzugsweise 830 bis 870 nm, beträgt.

7. Vorrichtung nach einem der Ansprüche 1 bis 6,
**dadurch gekennzeichnet, dass**
die Lichtquelle (6) eine Leistung von 20 bis 40 mW aufweist.

8. Vorrichtung nach einem der Ansprüche 1 bis 7,
**dadurch gekennzeichnet, dass**
die Verfahreinrichtung (8) eine Verfahrgeschwindigkeit von 1 bis 3 m/min, vorzugsweise 1,5 bis 2,5 m/min, aufweist.

9. Verfahren zum Betreiben einer Vorrichtung nach einem der Ansprüche 1 bis 8, wobei die Steuereinrichtung (10) die Lichtquelle (6) und/oder die Ausgabeeinrichtung (7) und/oder die Verfahreinrichtung (8) derart steuert, dass die auf die Hände (2) ausgegebene Intensität und/oder Dosis der Lichtstrahlung eine festgelegte Obergrenze nicht überschreitet,
**dadurch gekennzeichnet, dass**
die Steuereinrichtung (10) die Verfahrgeschwindigkeit der Verfahreinrichtung (8) in Abhängigkeit von der festgelegten Obergrenze einstellt.

10. Verfahren zum Betreiben einer Vorrichtung nach einem der Ansprüche 1 bis 8, wobei die Steuereinrichtung (10) die Lichtquelle (6) und/oder die Ausgabeeinrichtung (7) und/oder die Verfahreinrichtung (8) derart steuert, dass die auf die Hände (2) ausgegebene Intensität und/oder Dosis der Lichtstrahlung eine festgelegte Obergrenze nicht überschreitet,
**dadurch gekennzeichnet, dass**
der Abstand der wenigstens einen Hand (2) von der wenigstens einen Ausgabeeinrichtung (7) mittels einer Messeinrichtung gemessen wird, und dass die Steuereinrichtung (10) die Leistung der Lichtquelle (6) in Abhängigkeit von dem gemessenen Abstand einstellt.

11. Verfahren zum Betreiben einer Vorrichtung nach einem der Ansprüche 1 bis 8, wobei die Steuereinrichtung (10) die Lichtquelle (6) und/oder die Ausgabeeinrichtung (7) und/oder die Verfahreinrichtung (8) derart steuert, dass die auf die Hände (2) ausgegebene Intensität und/oder Dosis der Lichtstrahlung eine festgelegte Obergrenze nicht überschreitet,
**dadurch gekennzeichnet, dass**
die Steuereinrichtung (10) die Verfahreinrichtung (8) in Abhängigkeit einer mittels einer Sensoreinrichtung (11) ermittelten Position der Hände (2) derart steuert, dass Änderungen der Position der Hände (2) durch Verfahren der Ausgabeeinrichtung (7) mittels der Verfahreinrichtung (8) ausgeglichen werden.

12. Verfahren nach einem der Ansprüche 9, 10 oder 11,
**dadurch gekennzeichnet, dass**
die Steuereinrichtung (10) die Leistung der Lichtquelle (6) in Abhängigkeit von der festgelegten Obergrenze einstellt.

13. Verfahren nach einem der Ansprüche 9 bis 12,
**dadurch gekennzeichnet, dass**
die Steuereinrichtung (10) die Ausgabe der Lichtstrahlung durch die Ausgabeeinrichtung (7) in Abhängigkeit von der festgelegten Obergrenze zeitlich begrenzt.

## Claims

1. Apparatus (1) for disinfecting the hands (2) of a person, having a housing (3) which has a cavity (4) suitable for receiving at least one hand (2), at least one light source (6) for generating a focussed light radiation in the UV and/or IR range, at least one output device (7) operatively connected to the light source (6) for dispensing the light radiation into the cavity (4), at least one traversing device (8) for moving the light radiation and at least one control device (10) for controlling the light source (6) and/or the output device (7) and/or the traversing device (8), wherein the control device (10) is provided for controlling the at least one light source (6) and/or the at least one output device (7) and/or the at least one traversing device (8) in such a way that the intensity and/or dose of the light radiation output to the hands (2) does not exceed a fixed upper limit,
**characterised in that**
the control device (10) is provided for adjusting the traversing speed of the traversing device (8) as a function of the fixed upper limit, **in that** the distance of the at least one hand (2) from the at least one output device (7) can be measured by means of a measuring device, and **in that** the control device (10) is provided for adjusting the power of the light source (6) as a function of the measured distance.

2. Apparatus (1) for disinfecting the hands (2) of a person, having a housing (3) which has a cavity (4) suitable for receiving at least one hand (2), at least one light source (6) for generating a focussed light radiation in the UV and/or IR range, at least one output device (7) operatively connected to the light source (6) for dispensing the light radiation into the cavity (4), at least one traversing device (8) for moving the light radiation and at least one control device (10) for controlling the light source (6) and/or the output device (7) and/or the traversing device (8), wherein the control device (10) is provided for controlling the at least one light source (6) and/or the at least one output device (7) and/or the at least one traversing device (8) in such a way that the intensity and/or dose of the light radiation output to the hands (2) does not exceed a fixed upper limit,
**characterised by**
a sensor device (11) for monitoring the position of the hands (2), which is operatively connected to the control device (10) in order to forward the respective position of the hands (2) to the control device (10), wherein the control device (10) is provided for controlling the traversing device (8) in such a way that changes in the position of the hands (2) are compensated by moving the output device (7) by means of the traversing device (8).

3. Apparatus according to claim 1 or 2,
**characterised in that**
the traversing device (8) has at least one guide element (9) coupled to the at least one output device (7).

4. Apparatus according to claim 1, 2 or 3,
**characterised in that**
two output devices (7) are provided on opposite sides of the housing (3).

5. Apparatus according to one of claims 1 to 4,
**characterised in that**
the housing (3) is closed on all sides with the exception of an opening (5) for inserting at least one hand (2).

6. Apparatus according to one of claims 1 to 5,
**characterised in that**
the wavelength of the light radiation is 210 to 230 nm, preferably 215 to 225 nm, and/or 800 to 900 nm, preferably 830 to 870 nm.

7. Apparatus according to one of claims 1 to 6,
**characterised in that**
the light source (6) has a power of 20 to 40 mW.

8. Apparatus according to one of claims 1 to 7,
**characterised in that**
the traversing device (8) has a traversing speed of 1 to 3 m/min, preferably 1.5 to 2.5 m/min.

9. Method for operating an apparatus according to one of claims 1 to 8, wherein the control device (10) controls the light source (6) and/or the output device (7) and/or the traversing device (8) in such a way that the intensity and/or dose of the light radiation output to the hands (2) does not exceed a fixed upper limit,
**characterised in that**
the control device (10) adjusts the traversing speed of the traversing device (8) as a function of the fixed upper limit.

10. Method for operating an apparatus according to one of claims 1 to 8, wherein the control device (10) controls the light source (6) and/or the output device (7) and/or the traversing device (8) in such a way that the intensity and/or dose of the light radiation output to the hands (2) does not exceed a fixed upper limit,
**characterised in that**
the distance of the at least one hand (2) from the at least one output device (7) is measured by means of a measuring device, and **in that** the control device (10) adjusts the power of the light source (6) as a function of the measured distance.

11. Method for operating an apparatus according to one of claims 1 to 8, wherein the control device (10) controls the light source (6) and/or the output device (7) and/or the traversing device (8) in such a way that the intensity and/or dose of the light radiation output to the hands (2) does not exceed a fixed upper limit,
**characterised in that**
the control device (10) controls the traversing device (8) as a function of a position of the hands (2) determined by means of a sensor device (11) in such a way that changes in the position of the hands (2) are compensated by moving the output device (7) by means of the traversing device (8).

12. Method according to one of claims 9, 10 or 11,
**characterised in that**
the control device (10) adjusts the power of the light source (6) as a function of the fixed upper limit.

13. Method according to one of claims 9 to 12,
**characterised in that**
the control device (10) temporally limits the output of the light radiation by the output device (7) as a function of the fixed upper limit.

## Revendications

1. Dispositif (1) pour la désinfection des mains (2) d'une personne, avec un boîtier (3) qui comporte un espace creux (4) approprié pour recevoir au moins une main (2), avec au moins une source lumineuse (6) pour générer un faisceau lumineux focalisé sur la plage UV et/ou IR, au moins un dispositif d'émission (7) en liaison active avec la source lumineuse (6) pour émettre le faisceau lumineux dans l'espace creux (4), avec au moins un dispositif de déplacement (8) pour déplacer le faisceau lumineux et avec au moins un dispositif de commande (10) pour contrôler la source lumineuse (6) et/ou le dispositif d'émission (7) et/ou le dispositif de déplacement (8), lequel dispositif de commande (10) est prévu pour contrôler l'au moins une source lumineuse (6) et/ou l'au moins un dispositif d'émission (7) et/ou l'au moins un dispositif de déplacement (8) de telle sorte que l'intensité et/ou la dose émises du faisceau lumineux sur les mains (2) n'excèdent pas une limite supérieure définie,
**caractérisé en ce que**
le dispositif de commande (10) est prévu pour régler la vitesse de déplacement du dispositif de déplacement (8) en fonction de la limite supérieure définie, que la distance de l'au moins une main (2) par rapport à l'au moins un dispositif d'émission (7) peut être mesurée au moyen d'un dispositif de mesure et que le dispositif de commande (10) est prévu pour régler la puissance de la source lumineuse (6) en fonction de la distance mesurée.

2. Dispositif (1) pour la désinfection des mains (2) d'une personne, avec un boîtier (3) qui comporte un espace creux (4) approprié pour recevoir au moins une main (2), avec au moins une source lumineuse (6) pour générer un faisceau lumineux focalisé sur la plage UV et/ou IR, au moins un dispositif d'émission (7) en liaison active avec la source lumineuse (6) pour émettre le faisceau lumineux dans l'espace creux (4), avec au moins un dispositif de déplacement (8) pour déplacer le faisceau lumineux et avec au moins un dispositif de commande (10) pour contrôler la source lumineuse (6) et/ou le dispositif d'émission (7) et/ou le dispositif de déplacement (8), lequel dispositif de commande (10) est prévu pour contrôler l'au moins une source lumineuse (6) et/ou l'au moins un dispositif d'émission (7) et/ou l'au moins un dispositif de déplacement (8) de telle sorte que l'intensité et/ou la dose émises du faisceau lumineux sur les mains (2) n'excèdent pas une limite supérieure définie,
**caractérisé par**
un dispositif de détection (11) pour surveiller la position des mains (2) qui est en liaison active avec le dispositif de commande (10) pour transmettre la position des mains (2) au dispositif de commande (10), lequel dispositif de commande (10) est prévu contrôler le dispositif de déplacement (8) de telle sorte que les changements de position des mains (2) sont compensés par des déplacements du dispositif d'émission (7) au moyen du dispositif de déplacement (8).

3. Dispositif selon la revendication 1 ou 2,
**caractérisé en ce que**
le dispositif de déplacement (8) comporte au moins un dispositif de guidage (9) couplé avec l'au moins un dispositif d'émission (7).

4. Dispositif selon la revendication 1, 2 ou 3,
**caractérisé en ce que**
deux dispositifs d'émission (7) sont prévus sur des côtés opposés du boîtier (3).

5. Dispositif selon une des revendications 1 à 4,
**caractérisé en ce que**
le boîtier (3) est clos de tous les côtés à l'exception d'une ouverture (5) pour introduire au moins une main (2).

6. Dispositif selon une des revendications 1 à 5,
**caractérisé en ce que**
la longueur d'onde du faisceau lumineux est de 210 à 230 nm, de préférence de 215 à 225 nm, et/ou de 800 à 900 nm, de préférence de 830 à 870 nm.

7. Dispositif selon une des revendications 1 à 6,
**caractérisé en ce que**
la source lumineuse (6) présente une puissance de 20 à 40 mW.

8. Dispositif selon une des revendications 1 à 7,
**caractérisé en ce que**
le dispositif de déplacement (8) présente une vitesse de déplacement de 1 à 3 m/min, de préférence de 1,5 à 2,5 m/min.

9. Procédé de mise en oeuvre d'un dispositif selon une des revendications 1 à 8, selon lequel le dispositif de commande (10) contrôle la source lumineuse (6) et/ou le dispositif d'émission (7) et/ou le dispositif de déplacement (8) de telle sorte que l'intensité ou la dose du faisceau lumineux émise sur les mains (2) ne dépasse pas une limite supérieure définie,
**caractérisé en ce que**
le dispositif de commande (10) règle la vitesse de déplacement du dispositif de déplacement (8) en fonction de la limite supérieure définie.

10. Procédé de mise en oeuvre d'un dispositif selon une des revendications 1 à 8, selon lequel le dispositif de commande (10) contrôle la source lumineuse (6) et/ou le dispositif d'émission (7) et/ou le dispositif de déplacement (8) de telle sorte que l'intensité ou la dose du faisceau lumineux émise sur les mains (2) ne dépasse pas une limite supérieure définie,
**caractérisé en ce que**
la distance de l'au moins une main (2) par rapport à l'au moins un dispositif de d'émission (7) est mesurée au moyen d'un dispositif de mesure et que le dispositif de commande (10) règle la puissance de la source lumineuse (6) en fonction de la distance mesurée.

11. Procédé de mise en oeuvre d'un dispositif selon une des revendications 1 à 8, selon lequel le dispositif de commande (10) contrôle la source lumineuse (6) et/ou le dispositif d'émission (7) et/ou le dispositif de déplacement (8) de telle sorte que l'intensité ou la dose du faisceau lumineux émise sur les mains (2) ne dépasse pas une limite supérieure définie,
**caractérisé en ce que**
le dispositif de commande (10) contrôle le dispositif de déplacement (8) en fonction d'une position déterminée au moyen d'un dispositif de détection (11) de telle sorte que les changements de position des mains (2) sont compensés par des déplacements du dispositif d'émission (7) au moyen du dispositif de déplacement (8).

12. Procédé selon une des revendications 9, 10 et 11,
**caractérisé en ce que**
le dispositif de commande (10) règle la puissance de la source lumineuse (6) en fonction de la limite supérieure définie.

13. Procédé selon une des revendications 9 à 12,
**caractérisé en ce que**
le dispositif de commande (10) limite l'émission du faisceau lumineux par le dispositif d'émission (7) en fonction de la limite supérieure définie.
